# EUROPÄISCHE PATENTANMELDUNG

(11) **EP 2 446 917 A1**
(43) Veröffentlichungstag der Anmeldung: **02.05.2012**
(21) Anmeldenummer: 11184592.1
(22) Anmeldetag: 11.10.2011
(51) Int. Cl.: A61M 25/10

(54) **Katheter, System zum Einbringen einer intraluminalen Endoprothese sowie Verfahren zur Herstellung derselben**

(30) Priorität: 28.10.2010 US 407478 P
(71) Anmelder: Biotronik AG, 8180 Bülach (CH)
(72) Erfinder: Diener, Tobias, 90763 Fürth (DE); Burean, Elisabeta, 91054 Erlangen (DE); Fringes, Matthias, 91522 Ansbach (DE)
(74) Vertreter: Lindner-Vogt, Karin L.

(57) **Zusammenfassung**

Die vorliegende Erfindung betrifft einen Katheter mit einem Ballon (10,30) und mindestens einer pharmazeutisch aktiven Substanz. Um die pharmazeutisch aktive Substanz genauer dosieren zu können, ist die pharmazeutisch aktive Substanz in einem Reservoir (15, 35) innerhalb des Ballons (10,30) oder auf dem Ballon (10,30) angeordnet, wobei eine das Reservoir nach außen abschließende Wand (14,32,34) mindestens eine Sollbruchstelle (16) aufweist. Es werden ferner ein System zum Einbringen einer intraluminalen Endoprothese, vorzugsweise eines Stents, in einen Körperhohlraum sowie einfache und kostengünstige Verfahren zur Herstellung des Katheters bzw. des Systems beschrieben.

## Beschreibung

Die vorliegende Erfindung betrifft einen Katheter mit einem Ballon und mindestens einer pharmazeutisch aktiven Substanz. Die Erfindung betrifft ferner ein System zum Einbringen einer intraluminalen Endoprothese, vorzugsweise eines Stents, in einen Körperhohlraum bestehend aus der intraluminalen Endoprothese und einem solchen Katheter. Außerdem betrifft die Erfindung ein Verfahren zur Herstellung eines Katheters und ein Verfahren zur Herstellung eines Systems zum Einbringen einer intraluminalen Endoprothese.

Katheter sind Röhrchen oder Schläuche verschiedenen Durchmessers, die in den jeweiligen zu behandelnden Körperhohlraum eingeführt werden können. Sogenannte Ballonkatheter werden vor allem in der Angioplastie zur Erweiterung oder Wiedereröffnung eines Gefäßes eingesetzt. Ein solcher Ballonkatheter besteht aus einem Schlauch, der in einem vorgegebenen Bereich entlang des Schlauchs einen zunächst nicht dilatierten Ballon aufweist. Bei der Behandlung mit einem Ballonkatheter wird zuerst ein Führungsdraht in das zu behandelnde Gefäß eingeschoben. Anschließend wird der Ballonkatheter entlang des Führungsdrahts bis zu der zu behandelnden Stelle des Gefäßes vorgeschoben, so dass der Ballon im Bereich der zu behandelnden Stelle des Gefäßes platziert ist, die z.B. eine Stenose aufweist. Danach wird der Ballon dilatiert, d.h. entfaltet und/oder aufgedehnt, so dass die zu behandelnde Stelle wiedereröffnet oder erweitert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen. Durch die Erweiterung bzw. Wiedereröffnung des Gefäßes wird der Strom der Körperflüssigkeit in dem Gefäß nicht mehr oder nicht mehr in dem zuvor vorhandenen Maße behindert. Derartige Ballonkatheter können zusätzlich auch dazu verwendet werden, intraluminale Endoprothesen an eine zu behandelnde Stelle in einem Körperhohlraum einzubringen.

Intraluminale Endoprothesen, insbesondere in Form von Stents, weisen häufig ein rohrförmiges oder hohlzylinderförmiges Grundgitter auf, das an beiden Längsenden offen ist. Eine derartige Endoprothese wird bevorzugt mittels eines Katheters in den zu behandelnden Körperhohlraum eingesetzt und mittels des an dem Katheter angeordneten Ballons dilatiert. Selbstexpandierbare Stents (z.B. Nitinol-Stents) werden mit dem Katheter an die zu behandelnde Gefäßstelle transportiert und dort freigesetzt. Nach Entfernen des Katheters dient die Endoprothese dazu, den Körperhohlraum zu stützen. Hierbei haben Stents sich insbesondere zur Behandlung von Gefäßerkrankungen etabliert. Durch den Einsatz von Stents können verengte Bereiche in den Gefäßen dauerhaft so erweitert werden, dass ein Lumengewinn entsteht.

Während und/oder nach der Behandlung mit einem Katheter und/oder einem System zum Einbringen einer intraluminalen Endoprothese werden Medikamente (im Folgenden auch als pharmazeutisch aktive Substanz bezeichnet) verabreicht, welche die Behandlung mit dem Katheter oder der intraluminalen Endoprothese unterstützen sollen. Diese pharmazeutisch aktiven Substanzen sollen vor allem eine Inflammation des Gewebes oder einen erneuten Verschluss des Körperhohlraums (Restenose) verhindern.

Gegenwärtig werden Katheter mit Ballons eingesetzt und getestet, welche außen auf dem Ballon eine Beschichtung mit einer pharmazeutisch aktiven Substanz aufweisen. Hierdurch wird eine Einmalgabe des Medikaments ermöglicht, durch welche lediglich eine Versorgung des umgebenden Gewebes während des geöffneten Zustands des Ballons bewirkt wird. Das Medikament wirkt zudem nur an der Gewebeoberfläche.

Bei den bekannten Kathetern bzw. Systemen zum Einbringen einer intraluminalen Endoprothese besteht häufig das Problem, dass eine Medikamentenbeschichtung der beschriebenen Art mechanisch instabil ist. Beispielsweise geht während der Implantation durch Abschaben oder durch frühzeitiges Auswaschen des Wirkstoffs eine große Menge der pharmazeutisch aktiven Substanz verloren, so dass sich Partikel bilden können, welche im schlimmsten Fall zum Verschluss des behandelten Gefäßes führen können. Ferner wird durch die mechanische Instabilität der Beschichtung auch die genaue Dosierung des Wirkstoffes erschwert. Weitere, limitierende Faktoren bei den bisherigen Lösungen sind die Diffusionsgeschwindigkeit des Medikaments in das Gewebe, bzw. dessen lipophiler Charakter, sowie die Öffnungszeit des Ballons, welche ebenfalls eine ungenaue Dosierung des Wirkstoffs bedingen.

Es besteht daher die Aufgabe, einen Katheter zu schaffen, welcher eine gezielte und exakte Dosierung einer pharmazeutisch aktiven Substanz ermöglicht. Die Aufgabe besteht ferner darin, ein entsprechendes System aus Katheter und intraluminaler Endoprothese zu schaffen. Außerdem besteht die Aufgabe darin, Verfahren zur Herstellung eines Katheters bzw. eines Systems aus Katheter und intraluminaler Endoprothese anzugeben, welche eine kostengünstige Herstellung dieser Gegenstände ermöglichen.

Die obige Aufgabe wird durch einen Katheter gelöst, bei dem die pharmazeutisch aktive Substanz in einem Reservoir innerhalb des Ballons oder auf dem Ballon angeordnet ist, wobei eine das Reservoir nach außen abschließende Wand mindestens eine Sollbruchstelle aufweist.

Die mindestens eine Sollbruchstelle des Reservoirs ist in der Wand des Reservoirs vorgesehen, welche in Richtung der vorgesehenen Abgabe der pharmazeutisch aktiven Substanz liegt. Die Sollbruchstelle ist während des Handlings des Katheters außerhalb des zu behandelnden Körpers und während des Einbringens des Katheters zu dem Bereich des Körpers, an dem die Behandlung stattfinden soll, verschlossen. Es dringt während dieser Zeit demnach keine pharmazeutisch aktive Substanz aus dem Reservoir aus. Sobald der Ballon dilatiert wird und der Dilatationsdruck auch auf das Reservoir wirkt, platzt oder reißt die Sollbruchstelle auf und die pharmazeutisch aktive Substanz wird, ebenfalls unter einem Überdruck, insbesondere aufgrund des Dilatationsdrucks und das Anpressen der Ballonaußenseite an die Gefäßwand bzw. durch den bei der Dilatation kleiner werdenden Raum des Reservoirs, nach außen in Richtung des zu behandelnden Gewebes transportiert.

Der Vorteil des erfindungsgemäßen Katheters besteht darin, dass die pharmazeutisch aktiven Substanz exakt dosiert werden kann, da die pharmazeutisch aktive Substanz nicht als eine Beschichtung auf der Oberfläche sondern in einem zunächst abgeschlossenen Reservoir enthalten ist. Somit können die Wirkstoffe nicht während der Implantation mechanisch von der Oberfläche des Ballons gelöst werden. Durch Eröffnung der Sollbruchstelle während der Dilatation des Ballons wird eine gezielte "Injektion" der pharmazeutisch aktiven Substanz in das umgebende Gewebe des behandelten Körperhohlraums bewirkt. Hierdurch können als pharmazeutisch aktive Substanzen neben lipophilen Materialien auch Materialen eingesetzt werden, welche einen hydrophilen Charakter aufweisen. Die pharmazeutisch aktive Substanz wird durch den für die Dilatation des Ballons anliegenden Dilatationsdrucks (2 bis 30 bar, vorzugsweise 10 bis 18 bar) freigesetzt. Der anliegende Dilatationsdruck verursacht zumindest indirekt eine Eröffnung (z.B. Aufreißen oder Aufplatzen) der Sollbruchstelle. Hierdurch wird die pharmazeutisch aktive Substanz mit einem Überdruck aus dem Reservoir durch die Sollbruchstelle befördert und in das umgebende Gewebe injiziert, sodass kontrolliert bestimmte Tiefen des Gewebes erreicht werden können. Ein Abspülen der Medikamente von der Gewebeoberfläche nach dem Deflatieren des Ballons ist somit nicht mehr möglich.

Bei einer bekannten äußeren Beschichtung des Ballons mit einer pharmazeutisch aktiven Substanz wurde die pharmazeutisch aktive Substanz lediglich in dem kurzen Zeitraum, in dem der Ballon den behandelten Körperhohlraum auskleidet, zu dem betroffenen Gewebe übertragen. Demgegenüber erfolgt die Injektion der pharmazeutisch aktiven Substanz bei dem erfindungsgemäßen Katheter über einen längeren Zeitraum, nämlich über die gesamte Zeit, in der der Dilatationsdruck an dem Ballon anliegt. Durch Einstellung des Zeitraums, in dem der Dilatationsdruck an dem Ballon anliegt, kann zudem die ins Gewebe abgegebene Menge Medikament geregelt werden.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen, wirkt entzündungshemmend bzw. dient dem Unschädlichmachen von maladen Zellen in der Onkologie.

Derartige pharmazeutisch aktive Substanzen weisen beispielsweise eine antünflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe der Taxole oder Taxane, hier vorzugsweise Paclitaxel oder Sirolimus, sowie der Klasse der Limus-Medikamente bestehen.

Bei der vorliegenden Erfindung können in dem Reservoir eine oder eine Vielzahl von pharmazeutisch aktiven Substanzen angeordnet werden. Es ist ferner möglich, eine oder mehrere der pharmazeutisch aktiven Substanzen in Mikrokugeln oder Mikrokapseln anzuordnen, welche eine verzögerte (retardierte) Freisetzung der jeweiligen pharmazeutisch aktiven Substanz bewirken. In diesem Fall werden bei Eröffnung der Sollbruchstelle die Mikrokugeln oder Mikrokapseln in das zu behandelnde Gewebe freigesetzt.

Eine besonders einfache Realisierung eines entsprechenden Reservoirs für eine pharmazeutisch aktive Substanz wird dadurch erreicht, dass der Ballon eine Innenwand und eine Außenwand aufweist und dass das Reservoir zwischen der Innenwand und der Außenwand angeordnet ist.

Eine weitere einfache Möglichkeit der Verwirklichung eines Reservoirs für eine pharmazeutisch aktive Substanz besteht darin, mittels einer Taschenwand eine Tasche als Reservoir auszubilden, wobei diese auf der Innenseite oder der Außenseite einer Ballonwand angeordnet werden kann. Hierbei ist die Taschenwand entlang ihres gesamten Umfangs mittels eines Fügeverfahrens, vorzugsweise mittels Schweißen oder Kleben, mit der Ballonwand verbunden. Durch eine solche Gestaltung eines Reservoirs für eine pharmazeutisch aktive Substanz ist es insbesondere in vorteilhafter Weise möglich, Reservoire für eine pharmazeutisch aktive Substanz an beliebigen, vorgegebenen Stellen im Bereich des Ballons auch nachträglich anzubringen. Wird hierbei die Tasche an der Innenseite (luminale Seite) der Ballonwand angebracht, so ist die Sollbruchstelle in der Ballonwand im Bereich der Tasche angeordnet. Ist die Tasche mit dem Reservoir der pharmazeutisch aktiven Substanz an der Außenseite oder abluminalen Seite der Ballonwand vorgesehen, so wird die mindestens eine Sollbruchstelle in der Taschenwand angeordnet.

Die Verbindung von Taschenwand und Ballonwand erfolgt vorzugsweise mittels Schweißen oder Kleben, so dass die Taschenwand fest und mit einer dichten Verbindung mit der Ballonwand verbunden ist. Die Fügestelle zwischen Ballonwand und Taschenwand kann in einem Ausführungsbeispiel auch als Sollbruchstelle verwendet werden. In diesem Fall reißt die Fügestelle während der Dilatation des Ballons auf und wird somit undicht.

In einer bevorzugten Weiterbildung der Erfindung ist es vorgesehen, dass die Sollbruchstelle punktförmig oder linienförmig ausgebildet ist. Besonders bevorzugt ist in einer Ballonwand oder einer Taschenwand eine Vielzahl von Sollbruchstellen im Bereich des jeweiligen Reservoirs angeordnet. Durch die Wahl der Form der Sollbruchstelle wird auch das Endladeverhalten der pharmazeutisch aktiven Substanz und der Druck, mit dem diese in das Gewebe injiziert wird, bestimmt. Insbesondere bei einer punktförmigen Sollbruchstelle kann ein hoher Endladedruck und somit eine große Eindringtiefe in das Gewebe erreicht werden, da die Sollbruchstelle wie eine Düse wirkt. Eine bevorzugte Dichte der punktförmigen Sollbruchstellen, welche vorzugsweise regelmäßig über die jeweilige Wand verteilt im Bereich des Reservoirs angeordnet sind, beträgt 1 bis 500 Sollbruchstellen pro cm². Eine linienförmige Sollbruchstelle kann in einem Ausführungsbeispiel ring- oder spiralförmig, d.h. ring- oder spiralförmig um den zylinderförmigen Ballon umlaufend, ausgebildet sein. Linienförmige Sollbruchstellen haben vorzugsweise einen lateralen Abstand von 250 µm bis 5 mm, besonders bevorzugt einen Abstand von 500 µm bis 3 mm.

Es ist ferner bevorzugt, dass die Ballonwand und/oder die Außenwand und/oder die Taschenwand im Bereich der Sollbruchstelle eine geringere Dicke als im übrigen Bereich der jeweiligen Wand, vorzugsweise eine Dicke von 5% bis 75%, besonders bevorzugt eine Dicke von 10% bis 25%, der Dicke der jeweiligen Wand im übrigen Bereich aufweist. Im Bereich der Sollbruchstelle eine dünnere Wand als im übrigen Bereich der jeweiligen Wand vorzusehen, ist eine besonders einfache Möglichkeit, eine Sollbruchstelle zu verwirklichen.

Eine weitere vorteilhafte Möglichkeit für die Realisierung einer Sollbruchstelle besteht darin, das Material der Ballonwand oder der Außenwand oder der Taschenwand im Bereich der Sollbruchstelle zu schwächen, wobei die Schwächung vorzugsweise mittels Laser, Ätzen, mit Materialabtragung (beispielsweise Schneiden, Perforieren), durch Einbringen einer Fügestelle und/oder Materialversprödung erzeugt ist. Die Materialversprödung kann z.B. während der Ballonhautherstellung durch wiederholtes, zyklisches Abknicken des Werkstoffs erzeugt werden. Der Bereich des spröden Materials wird an der Knick-oder Biegekante hergestellt. Hier liegen gegebenenfalls auch Mikrorisse vor.

Eine Sollbruchstelle kann ferner dadurch realisiert werden, dass die Ballonwand und/oder die Außenwand und/oder die Taschenwand im Bereich der Sollbruchstelle ein zu dem benachbarten, übrigen Bereich der jeweiligen Wand vorgesehenen Material unterschiedliches Material aufweist. Das Material, das im Bereich der Sollbruchstelle verwendet wird, ist vorzugsweise weicher oder spröder als das Material im übrigen Bereich der jeweiligen Wand. An der Sollbruchstelle kann z.B. das gleiche Polymer, das auch für die Taschenwand oder die Ballonwand verwendet wird, nur in lang- oder kurzkettigerer Form eingesetzt werden. Bereits bei der Herstellung der Taschen- oder Ballonwand, z.B. mittels Extrudieren, Spritzen etc., wird das Material der Sollbruchstelle mit dem Material der Taschenwand oder der Ballonwand zusammengefügt. Lang- und Kurzkettige Polymere der gleichen Klasse haben andere Festigkeiten und Materialeigenschaften. Alternativ können auch zwei Polymere mit unterschiedlichen Festigkeiten/Sprödigkeiten eingesetzt werden, die dann später verklebt oder verschweißt werden.

Als Material der Ballonwand bzw. Taschenwand können beispielsweise Polyethylene (PET), Polycarbonate (PC), Polyamide (PA), Polyimide (PI), PEBAX, PA11, PA12 oder PVC sowie deren Blends eingesetzt werden. Ferner können diese Materialien mit einer Kompoundierung/Verblendung mit der Polymerklasse der Silikone oder, allgemeiner, der thermoplastischen und/oder elastomeren Polymere versehen werden.

Die obige Aufgabe wird mit den oben angegebenen Vorteilen außerdem durch ein System zum Einbringen einer intraluminalen Endoprothese gelöst, bei dem die intraluminale Endoprothese außen auf dem Ballon angeordnet, vorzugsweise auf diesen aufgecrimpt, ist. In diesem Zustand ist der Ballon noch nicht dilatiert. Durch die Dilatation des Ballons des Katheters wird gleichzeitig die außen auf dem Ballon angeordnete intraluminale Endoprothese dauerhaft aufgedehnt und nach dem Deflatieren des Ballons verbleibt die intraluminale Endoprothese in dem jeweiligen Körperhohlraum.

In einem besonders bevorzugten Ausführungsbeispiel ist die intraluminale Endoprothese als biodegradierbarer Stent, vorzugsweise als AMS (absorbierbarer Metallstent) oder absorbierbarer Polymerstent, ausgebildet. Derartige Stents enthalten als Hauptbestandteil ein biodegradierbares Metall, vorzugsweise Magnesium, Eisen, Zink, Wolfram und/oder eine Legierung der genannten Metalle. Polymerstents werden aus degradierbaren Polymeren hergestellt.

Hierbei werden unter dem Begriff'Biodegradation' hydrolytische, enzymatische oder andere Stoffwechsel-bedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Biodegradierbare Materialien können aus degradierbaren Polymeren oder den angegebenen Metallen realisiert werden.

Die obige Aufgabe wird ferner durch ein Verfahren gelöst, bei dem zunächst ein Katheterkörper mit Innenschaft und ggf. einem Außenschaft bereit gestellt wird, anschließend eine Balloninnenwand und eine Ballonaußenwand hergestellt werden, die Balloninnenwand und die Ballonaußenwand nach Anordnung einer pharmazeutisch aktiven Substanz zwischen den beiden Wänden miteinander verbunden werden und danach der Ballon mit dem Innenschaft und ggf. auch mit dem Außenschaft verbunden wird. Mindestens eine Sollbruchstelle wird in die Außenwand des Ballons entweder während der Herstellung des Ballons oder nachträglich eingebracht.

Die Reservoir-Geometrie des nach dem obigen Verfahren hergestellten doppellumigen Ballons kann im einfachsten Fall als Hohlzylinder mit der Manteldicke M beschrieben werden, wobei M den Abstand zwischen der äußeren Oberfläche der Balloninnenwand und der inneren Oberfläche der Ballonaußenwand im Ballonmittelteil beinhaltet. Die Manteldicke M beträgt beispielsweise zwischen 50 µm und 1 mm, insbesondere zwischen 100 µm und 750 µm. Bei einer solchen Ausgestaltung sind ferner beispielsweise punktförmige Sollbruchstellen regelmäßig über die Ballonaußenwand verteilt angeordnet.

Die obige Aufgabe wird außerdem gelöst durch ein Verfahren, bei dem zunächst ein Katheterkörper mit Innenschaft und ggf. einem Außenschaft bereit gestellt wird, anschließend eine Ballonwand hergestellt wird, nun auf der Innenseite oder der Außenseite der Ballonwand eine Taschenwand zur Ausbildung einer Tasche als Reservoir für mindestens eine pharmazeutisch aktive Substanz angeordnet wird, wobei die Taschenwand nach Anordnung der mindestens einen pharmazeutisch aktiven Substanz zwischen Ballonwand und einer Taschenwand mit der Ballonwand verbunden wird, danach der Ballon mit dem Innenschaft und ggf. auch mit dem Außenschaft des Katheterkörpers verbunden wird, wobei mindestens eine Sollbruchstelle in die Ballonwand oder die Taschenwand eingebracht wird.

Die oben beschriebenen Verfahren sind einfache Verfahren, um einen vorteilhaften Katheter mit einem Reservoir, dessen nach außen abschließende Wand mindestens eine Sollbruchstelle aufweist, herzustellen.

Alternativ zu den oben beschriebenen Verfahren kann die mindestens eine pharmazeutisch aktive Substanz auch erst nachträglich, d.h. nach Fertigstellung des Ballons mit (leerem) Reservoir, in das jeweilige Reservoir z.B. durch Eindiffusion eingebracht werden.

Vorzugsweise werden die Balloninnenwand und die Ballonaußenwand und/oder die Ballonwand und die Taschenwand mittels eines Fügeverfahrens, vorzugsweise mittels Schweißen oder Kleben, miteinander verbunden.

Die mindestens eine Sollbruchstelle kann vorzugsweise durch Schwächung des Materials der Ballonwand und/oder der Taschenwand hergestellt werden, wobei die Schwächung mittels Laser, Ätzen, Materialabtragung, Einbringung einer Fügestelle und/oder Materialversprödung erzeugt wird.

Vorzugsweise sind Reservoir-Taschen mit der Taschenwand, auf der die pharmazeutisch aktive Substanz aufgetragen ist, und gegebenenfalls der mindestens einen Sollbruchstelle bereits vorgefertigt und werden nach Fertigstellung der Ballonwand lediglich innen oder außen auf die Ballonwand aufgeklebt oder mittels Schweißen mit der Ballonwand verbunden.

Ein nach solchen Verfahren hergestellter Katheter erlaubt eine einfache Handhabung eines mit einem Medikament versehenen Ballons ohne Sicherheitsrisiko für den Verwender. Durch die Anordnung der pharmazeutisch aktiven Substanz in einem Reservoir besteht nicht mehr die Möglichkeit, dass eine Medikamentenbeschichtung während einer Operation abplatzt und die Umgebung kontaminiert. Ferner können durch die Anordnung der pharmazeutisch aktiven Substanz in einem zunächst abgeschlossenen Reservoir eine Vielzahl von pharmazeutisch aktiven Substanzen verwendet werden, die für eine Beschichtung nicht zur Verfügung stehen. Zudem sind eine genauere Dosierung und eine exaktere Platzierung der pharmazeutisch aktiven Substanz in dem behandelten Körperhohlraum möglich.

Die obige Aufgabenstellung wird ferner durch ein einfaches und kostengünstiges Verfahren zur Herstellung eines Systems gelöst, bei dem zunächst der Katheter nach einer der vorstehend beschriebenen Möglichkeiten hergestellt wird und anschließend die intraluminale Endoprothese außen auf dem vorzugsweise gefalteten Ballon derart fest angeordnet wird, dass sie diesen mindestens teilweise umgibt. Besonders bevorzugt und in einer sehr einfachen Art und Weise wird die intraluminale Endoprothese auf dem Ballon mittels Aufcrimpen angeordnet.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: einen Längsschnitt eines ersten Ballons für einen erfindungsgemäßen Katheter im nicht dilatierten Zustand,
- Fig. 2: eine Ansicht des Ballons gemäß Figur 1 von der Seite,
- Fig. 3: einen Längsschnitt eines zweiten Ballons für einen erfindungsgemäßen Katheter im nicht dilatierten Zustand,
- Fig. 4: eine Ansicht des Ballons gemäß Figur 2 von der Seite,
- Fig. 5: einen Längsschnitt eines ersten Ausführungsbeispiels eines erfindungsgemäßen Katheters,
- Fig. 6: einen Längsschnitt eines zweiten Ausführungsbeispiels eines erfindungsgemäßen Katheters,

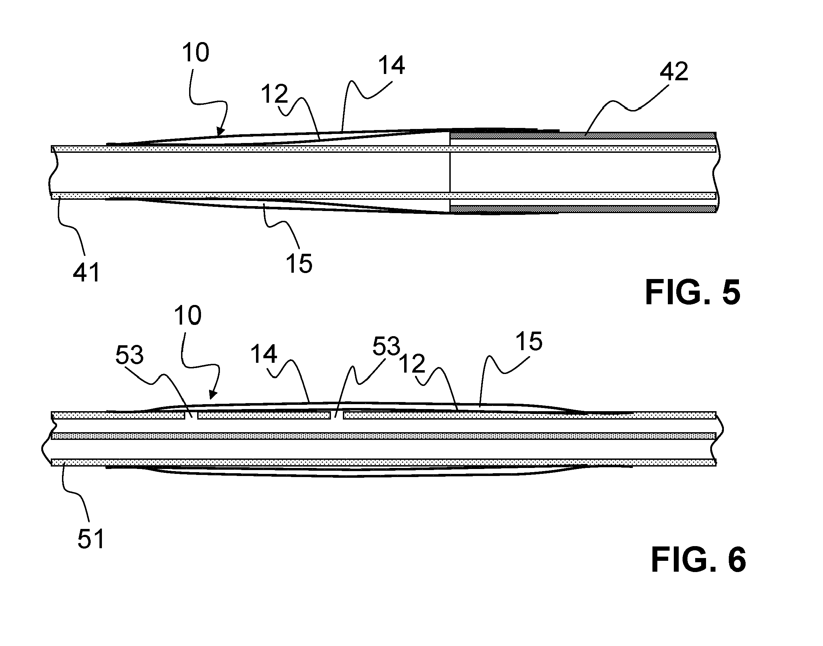

- Fig. 6: ein erstes Ausführungsbeispiel eines erfindungsgemäßen Systems in einer Ansicht von der Seite und

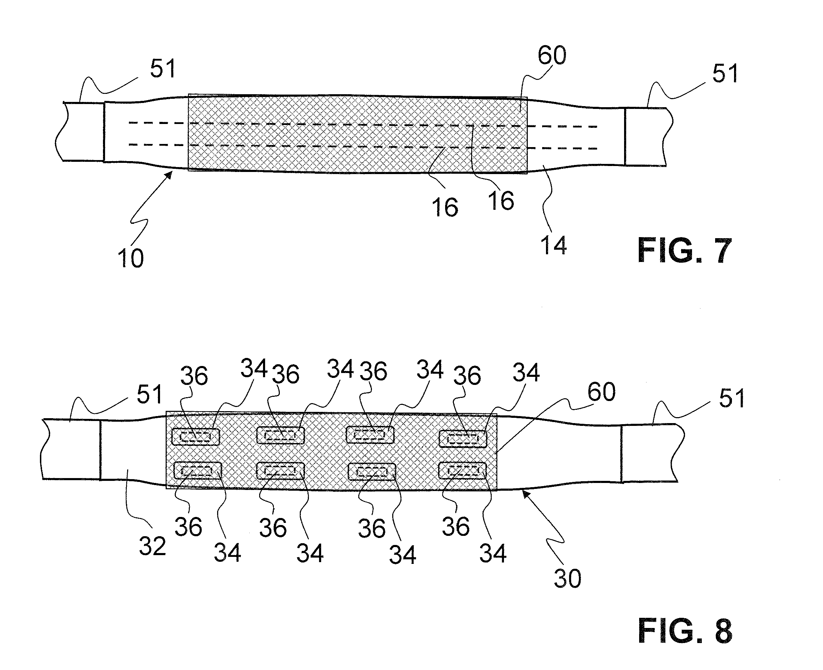

- Fig. 7: ein zweites Ausführungsbeispiel eines erfindungsgemäßen Systems in einer Ansicht von der Seite.

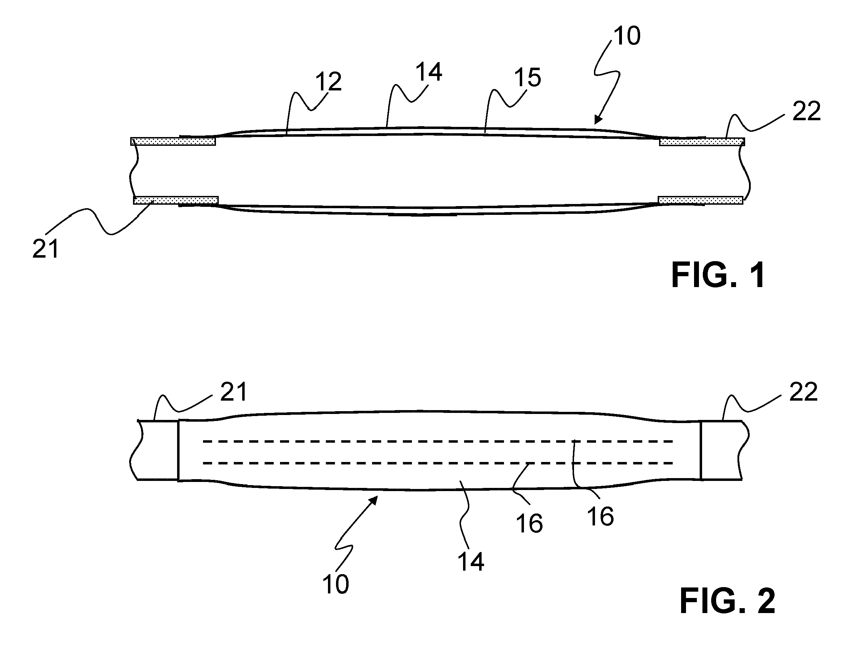

Figur 1 zeigt die Vorform eines Ballons 10 für einen erfindungsgemäßen Katheter, welcher mit seinem distalen Ende auf einen ersten Schaft 21 und seinem proximalen Ende auf einem zweiten Schaft 22 angeordnet ist. Der Ballon 10 weist eine Innenwand 12 und eine Außenwand 14 auf, welche an dem jeweiligen distalen bzw. proximalen Ende miteinander verbunden sind. Zwischen der Innenwand 12 und der Außenwand 14 wird ein Reservoir 15 ausgebildet, in dem mindestens eine (nicht dargestellte) pharmazeutisch aktive Substanz angeordnet ist. Als Materialien für Innenwand 12 und Außenwand 14 kommen alle gängigen Ballonmaterialien wie Polyethylene (PET), Polycarbonate (PC), Polyamide (PA), Polyimide (PI), PEBAX, PA11, PA12 oder PVC sowie deren Blends in Frage.

In der Außenwand 14 des Ballons 10 sind linienförmige Sollbruchstellen 16 eingebracht, welche beispielsweise mittels Laser hergestellt worden sind. Durch die mit dem Laser aufgebrachte thermische Energie wurde im Bereich der Linien 16 eine Schwächung des Materials erzeugt. Die Schwächung des Materials wirkt ähnlich wie ein Materialabtrag. Beim Aufbringen des Dilatationsdruckes, sucht sich der Druck den geringsten Widerstand. Das Reservoir wird daher an diesen Stellen bevorzugt aufreißen. Im Bereich der linienförmigen Sollbruchstelle 16 ist das Material der Außenwand 14 des Ballons 10 geschwächt. Beim Dilatieren des Ballons 10 würden die Sollbruchstellen 16 aufreißen und die in dem Reservoir 15 angeordnete pharmazeutisch aktive Substanz(en) freigeben. Die Sollbruchstellen 16 sind in Figur 2 mittels gestrichelter Linien gekennzeichnet.

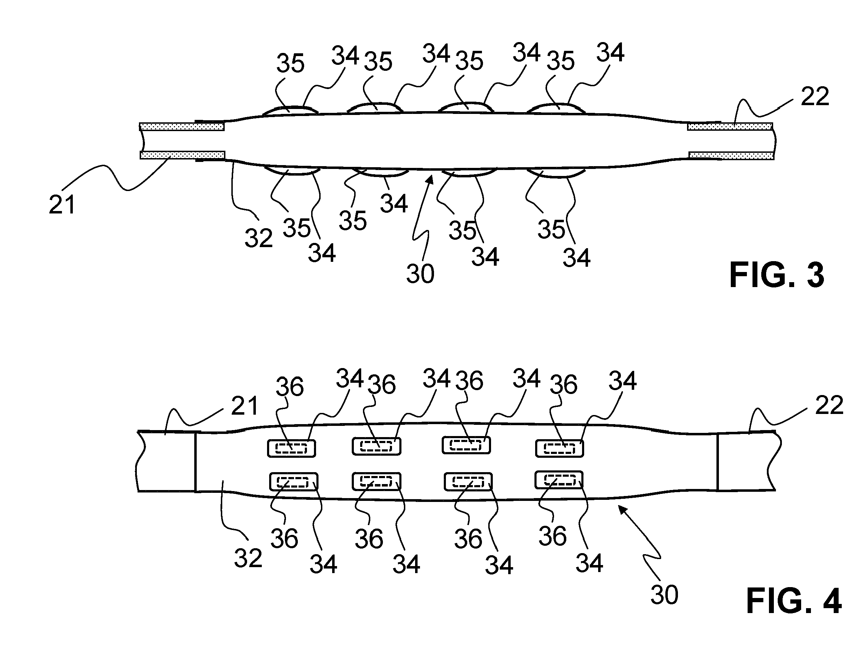

Figur 3 zeigt ein zweites Ausführungsbeispiel eines Ballons 30 für einen erfindungsgemäßen Katheter. Analog zu dem in Figur 1 dargestellten Ballon 10 ist der Ballon 30 mit seinem distalen Ende auf einem ersten Schaft 21 und seinem proximalen Ende auf einem zweiten Schaft 22 angeordnet. Der Ballon 30 weist eine Ballonwand 32 mit auf der Außenseite aufgesetzten Taschen auf. Jede Tasche besitzt eine Taschenaußenwand 34, welche zusammen mit der Ballonwand 32 das jeweilige Reservoir 35 für die pharmazeutisch aktive Substanz ausbildet. Die Taschenaußenwand 34 ist mit der Ballonwand 32 mittels Schweißen oder Kleben verbunden. Schweißverfahren werden bevorzugt mittels Laser durchgeführt. Hierbei wird die Oberfläche der zu verbindenden Teile angeschmolzen, ohne seine Materialeigenschaften zu verändern, und die Ballonwand 32 und die Taschenaußenwand 34 werden anschließend verbunden. Das Verkleben erfolgt mit geeigneten Klebemitteln und -verfahren. Zum Kaltverfügen können beispielsweise Klebstoffe der Epoxydoder Acrylmaterialklassen verwendet werden. Methodisch erfolgt das Verfügen dann über Anpressen beider Teile miteinander. Bei thermischen Fügeverfahren können alle thermoplastischen Polymerwerkstoffe eingesetzt werden, mit denen das jeweilige Ballonwandmaterial oder Taschenwandmaterial benetzbar sind. Hierbei wird zunächst die Fügestelle mit Klebstoff benetzt und anschließend bei Beaufschlagung mit Wärme (z.B. mittels eines IR-Strahlers) verpresst.

In jedem Reservoir 35 ist mindestens eine (nicht dargestellte) pharmazeutisch aktive Substanz, gegebenenfalls in Mikrokapseln, vorgesehen.

In Figur 4 ist gezeigt, dass jede Tasche mit der Außenwand 34 in dem ovalen Bereich 36 der Taschenaußenwand 34, welcher mit einer gestrichelten Linie umrandet ist, punktförmige Sollbruchstellen aufweist. In dem Bereich 36 sind die punktförmigen Sollbruchstellen regelmäßig verteilt angeordnet mit einer Dichte von 1 bis 500 Sollbruchstellen pro cm². Die punktförmigen Sollbruchstellen weisen die oben im Zusammenhang mit den linienförmigen Sollbruchstellen 16 dargestellten Eigenschaften auf. Insbesondere durchdringen die Sollbruchstellen den Werkstoff nicht komplett. Die Aufbringung der Sollbruchstellen kann über mechanische, chemische oder thermische Verfahren erreicht werden. Der Ballon wird bis zu diesem Schritt in herkömmlichen bekannten Verfahren hergestellt. Nach der Eröffnung der im Bereich 36 vorgesehenen Sollbruchstellen beim Dilatieren des Ballons 30 kann die pharmazeutisch aktive Substanz durch diese hindurch gelangen und in das (nicht dargestellte) umgebende Gewebe des behandelten Körperhohlraums injiziert werden.

Der erfindungsgemäße Katheter weist in dem in Figur 5 dargestellten ersten Ausführungsbeispiel einen Innenschaft 41 auf, mit dem der distale Ballonhals des Ballons 10 verbunden ist. Der proximale Ballonhals des Ballons 10 ist mit einem Außenschaft 42 des Katheters verbunden, wobei der Außenschaft 42 konzentrisch auf dem Innenschaft 41 angeordnet ist und diesen umgibt. Durch den zwischen Außenschaft 42 und Innenschaft 41 gebildeten hohlzylinderförmigen Hohlraum ist eine Inflation des Ballons 10 möglich, so dass dieser dilatiert wird. Alternativ zu dem in Figur 5 dargestellten Ausführungsbeispiel kann natürlich auch der anhand der Figuren 3 und 4 dargestellte Ballon 30 auf dem Innenschaft 41 bzw. Innenschaft 42 angeordnet sein.

Figur 6 zeigt ein zweites Ausführungsbeispiel eines erfindungsgemäßen Katheters mit einem zweilumigen Innenschaft 51. In diesem Ausführungsbeispiel ist sowohl der distale Ballonhals als auch der proximale Ballonhals des Ballons 10 mit dem Innenschaft 51 verbunden. Ein Lumen des zweilumigen Innenschafts 51 weist Öffnungen 53 auf, durch welche eine Inflation des Ballons 10 erfolgen kann. Auch auf einem solchen zweilumigen Innenschaft 51 kann der anhand der Figur 3 und 4 dargestellte Ballon 30 angeordnet werden.

Um ein erfindungsgemäßes System herzustellen, wird nun (siehe Figuren 7 und 8) nach Fertigstellung des jeweiligen Katheters eine intraluminale Endoprothese, beispielsweise ein Stent 60, auf den Ballon 10 bzw. den Ballon 30 aufgebracht. Vorzugsweise wird ein solcher Stent 60 auf einen Ballon 10 bzw. 30 aufgecrimpt. Bei der Behandlung wird dann der Stent 60 zusammen mit der Dilatation des Ballons 10 bzw. des Ballons 30 und dem Körperhohlraum aufgedehnt. Nach Deflatieren des jeweiligen Ballons 10, 30 verbleibt der Stent 60 in dem Körperhohlraum und stützt diesen.

Figur 7 zeigt den in Figur 6 dargestellten Katheter mit dem anhand der Figuren 1 und 2 beschriebenen Ballon 10, auf den Stent 60 aufgecrimpt ist. Entsprechend ist in Figur 8 der in Figur 6 dargestellte Katheter mit dem in den Figuren 3 und 4 gezeigten Ballon 30 und Stent 60 veranschaulicht.

### Bezugszeichenliste

- 10: Ballon
- 12: Innenwand des Ballons 10
- 14: Außenwand des Ballons 10
- 15: Reservoir
- 16: linienförmige Sollbruchstelle
- 21: erster Schaft
- 22: zweiter Schaft
- 30: Ballon
- 32: Ballonwand
- 34: Taschenaußenwand
- 35: Reservoir
- 36: Bereich, in dem punktförmige Sollbruchstellen angeordnet sind
- 41: Innenschaft
- 42: Außenschaft
- 51: Innenschaft
- 53: Öffnung
- 60: Stent

## Patentansprüche

1. Katheter mit einem Ballon (10,30) und mindestens einer pharmazeutisch aktiven Substanz, **dadurch gekennzeichnet, dass** die pharmazeutisch aktive Substanz in einem Reservoir (15, 35) innerhalb des Ballons (10,30) oder auf dem Ballon (10,30) angeordnet ist, wobei eine das Reservoir nach außen abschließende Wand (14,32,34) mindestens eine Sollbruchstelle (16) aufweist.

2. Katheter nach Anspruch 1, **dadurch gekennzeichnet, dass** der Ballon (10) eine Innenwand (12) und eine Außenwand (14) aufweist und das Reservoir (15) zwischen der Innenwand (12) und der Außenwand (14) ausgebildet ist.

3. Katheter nach einem der vorhergehenden Ansprüche, **dadurch gekennzeichnet, dass** auf der Innenseite oder der Außenseite einer Ballonwand (32) mittels einer Taschenwand (34) eine Tasche als Reservoir (35) ausgebildet ist, wobei die Taschenwand (34) entlang ihres gesamten Umfangs mittels eines Fügeverfahrens, vorzugsweise mittels Schweißen oder Kleben, mit der Ballonwand (32) verbunden ist.

4. Katheter nach einem der vorhergehenden Ansprüche, **dadurch gekennzeichnet, dass** die Sollbruchstelle (16) punktförmig oder linienförmig ausgebildet ist.

5. Katheter nach einem der vorhergehenden Ansprüche, **dadurch gekennzeichnet, dass** die Ballonwand (32) und/oder die Außenwand (14) und/oder die Taschenwand (34) im Bereich der Sollbruchstelle (16) eine geringere Dicke als im übrigen Bereich der jeweiligen Wand (32,14,34) aufweist, vorzugsweise eine Dicke von 5% bis 75%, besonders bevorzugt eine Dicke von 10% bis 25%, der Dicke der jeweiligen Wand (32,14,34) im übrigen Bereich.

6. Katheter nach einem der vorhergehenden Ansprüche, **dadurch gekennzeichnet, dass** das Material der Ballonwand (32) und/oder der Außenwand (14) und/oder der Taschenwand (34) im Bereich der Sollbruchstelle (16) geschwächt ist, wobei die Schwächung vorzugsweise mittels Laser, Ätzen, Materialabtragung, Einbringen einer Fügestelle und/oder Materialversprödung erzeugt ist.

7. Katheter nach einem der vorhergehenden Ansprüche, **dadurch gekennzeichnet, dass** die Ballonwand (32) und/oder die Außenwand (14) und/oder die Taschenwand (34) im Bereich der Sollbruchstelle ein zu dem benachbarten, übrigen Bereich der jeweiligen Wand vorgesehenen Material unterschiedliches Material aufweist.

8. Katheter nach einem der vorhergehenden Ansprüche, **dadurch gekennzeichnet, dass** die Dichte von punktförmigen Sollbruchstellen, welche vorzugsweise regelmäßig über die Ballonwand (32) und/oder die Außenwand (14) und/oder die Taschenwand (34) verteilt im Bereich des Reservoirs angeordnet sind, 1 bis 500 Sollbruchstellen pro cm² beträgt.

9. Katheter nach einem der vorhergehenden Ansprüche, **dadurch gekennzeichnet, dass** die linienförmigen Sollbruchstellen (16) einen lateralen Abstand von 250 µm bis 5 mm, vorzugsweise einen lateralen Abstand von 500 µm bis 3 mm, aufweisen.

10. System zum Einbringen einer intraluminalen Endoprothese (60), vorzugsweise eines Stents, in einen Körperhohlraum bestehend aus der intraluminalen Endoprothese (60) und einem Katheter nach einem der vorhergehenden Ansprüche, wobei die intraluminale Endoprothese (60) außen auf dem Ballon (10,30) angeordnet, vorzugsweise auf diesen aufgecrimpt, ist.

11. System nach Anspruch 10, **dadurch gekennzeichnet, dass** die intraluminale Endoprothese als biodegradierbarer Stent (60) ausgebildet ist.

12. Verfahren zur Herstellung eines Katheters, insbesondere nach einem der Ansprüche 1 bis 9, **dadurch gekennzeichnet, dass**
zunächst ein Katheterkörper mit Innenschaft (41,51) und gegebenenfalls einem Außenschaft bereit gestellt wird,
anschließend eine Balloninnenwand (12) und eine Ballonaußenwand (14) hergestellt werden, die Balloninnenwand (12) und die Ballonaußenwand (14) nach Anordnung einer pharmazeutisch aktiven Substanz zwischen den beiden Wänden (12,14) miteinander verbunden werden und danach der Ballon (10) mit dem Innenschaft (41,51) und gegebenenfalls auch mit dem Außenschaft (42) verbunden wird, wobei mindestens eine Sollbruchstelle (16) in die Außenwand des Ballons (10) eingebracht wird.

13. Verfahren zur Herstellung eines Katheters, insbesondere nach einem der Ansprüche 1 bis 9, **dadurch gekennzeichnet, dass**
zunächst ein Katheterkörper mit Innenschaft (41,51) und ggf. einem Außenschaft (42) bereit gestellt wird,
anschließend eine Ballonwand (32) hergestellt wird, nun auf der Innenseite oder der Außenseite der Ballonwand (32) eine Taschenwand (34) zur Ausbildung einer Tasche als Reservoir für mindestens eine pharmazeutisch aktive Substanz angeordnet wird, wobei die Taschenwand (34) nach Anordnung der mindestens einen pharmazeutisch aktiven Substanz zwischen Ballonwand (32) und einer Taschenwand (34) mit der Ballonwand (32) verbunden wird, danach der Ballon (30) mit dem Innenschaft (41,51) und gegebenenfalls auch mit dem Außenschaft (42) des Katheterkörpers verbunden wird, wobei mindestens eine Sollbruchstelle in die Ballonwand (32) oder die Taschenwand (34) eingebracht wird.

14. Verfahren nach einem der Ansprüche 12 oder 13, **dadurch gekennzeichnet, dass** die Balloninnenwand (12) und die Ballonaußenwand (14) und/oder die Ballonwand (32) und die Taschenwand (34) mittels eines Fügeverfahrens, vorzugsweise mittels Schweißen oder Kleben, miteinander verbunden werden.

15. Verfahren nach einem der Ansprüche 12 bis 14, **dadurch gekennzeichnet, dass** die Sollbruchstelle (16) punktförmig oder linienförmig ausgebildet ist.

16. Verfahren nach einem der Ansprüche 12 bis 15, **dadurch gekennzeichnet, dass** die Sollbruchstelle (16) durch Schwächung des Materials der Außenwand (14) und/oder der Ballonwand (32) und/oder der Taschenwand (34), vorzugsweise mit Hilfe von Laser, Ätzen, Materialabtragung, Einbringung einer Fügestelle und/oder Materialversprödung, erzeugt ist.

17. Verfahren nach einem der Ansprüche 12 bis 16, **dadurch gekennzeichnet, dass** die Ballonwand (32) und/oder die Außenwand (14) und/oder die Taschenwand (34) im Bereich der Sollbruchstelle (16) eine geringere Dicke als im übrigen Bereich der jeweiligen Wand (14,32,34) aufweist, vorzugsweise eine Dicke von 5% bis 75%, besonders bevorzugt eine Dicke von 10% bis 25%, der Dicke der jeweiligen Wand im übrigen Bereich.

18. Verfahren zur Herstellung eines Systems zum Einbringen einer intraluminalen Endoprothese (60), vorzugsweise eines Stents, in einen Körperhohlraum bestehend aus der intraluminalen Endoprothese (60) und einem Katheter, wobei zunächst der Katheter nach einem in den Ansprüchen 12 bis 17 beschriebenen Verfahren hergestellt wird und anschließend die intraluminale Endoprothese (60) außen auf dem vorzugsweise gefalteten Ballon (10,30) derart fest angeordnet wird, vorzugsweise auf diesen aufgecrimpt wird, dass sie diesen mindestens teilweise umgibt.
